# EUROPÄISCHE PATENTANMELDUNG

(11) **EP 1 295 861 A2**
(43) Veröffentlichungstag der Anmeldung: **26.03.2003**
(21) Anmeldenummer: 02020853.4
(22) Anmeldetag: 18.09.2002
(51) Int. Cl.: C07C 59/72, C02F 1/26

(54) **Calix(6)arene, Verfahren zu ihrer Herstellung und ihre Anwendung**

(30) Priorität: 24.09.2001 DE 10146928
(71) Anmelder: Synaptec GmbH, 17489 Greifswald (DE)
(72) Erfinder: Ludwig, Rainer, 10779 Berlin (DE)
(74) Vertreter: Meyerhöfer, Dietmar, Dipl.-Ing.

(57) **Zusammenfassung**

Erfindung betrifft Calix[6]arene, die alpha-halogenierte Carbonsäuregruppen enthalten, mit der Struktur 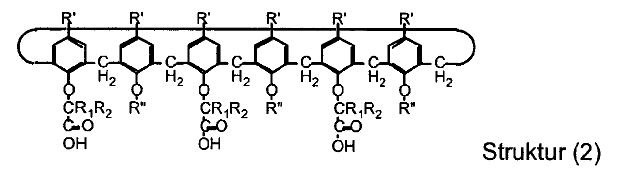 wobei R₁ = F, Cl oder Br, R₂ = H, F, Cl oder Br, R' = tertiär-Butyl, tertiär-Octyl, H, Aryl- oder eine andere Alkylgruppe, R" = H, Alkyl-, Aryl-, Alkylcarbonylmethoxy-, NHoder N, N-disubstituierte Carbamoylmethoxy-Gruppen sein können sowie ihre Herstellung und Anwendung.

Diese Calix[6]arene sind geeignet, dreiwertige Actiniden selektiv von dreiwertigen Lanthaniden und von Alkali- und Erdalkalisalzen abzutrennen und sie durch Rückextraktion aufkonzentriert und in reiner Form zu gewinnen, wobei die organische Verbindung in ihrer ursprünglichen Form zurückgewonnen wird.

Die Synthese geht von Phenol und Formaldehyd aus, das entstehende Calix[6]aren als Zwischenprodukt wird unter Ausnutzung von Templateffekten mit drei alphahalogenierten Carbonsäuregruppen derivatisiert. Weitere funktionelle Gruppen können an den unsubstituierten OH-Gruppen in das Molekül eingeführt werden.

Die Extraktion von Am³⁺ erfolgt bei deutlich tief] -Werten als dies bei Lanthaniden(III) der Fall ist. Damit wird eine chemische Trennung erreicht, die industriell ausgenutzt werden kann. Entscheidend für die gute Trennung Am³⁺/Ln³⁺ ist die Anwesenheit gemischter Funktionalitäten im Calix[6]aren-Molekül, einschließlich dreier saurer Gruppen. Entscheidend für die Abtrennung im sauren pH-Bereich ist die Anwesenheit von Halogen in den sauren Gruppen.

Die Calix[6]arene können in organischen Verdünnungsmitteln gelöst werden. Sie komplexieren beim Kontakt mit wäßrigen Lösungen dreiwertige Actinide, wenn diese darin bei pH < 4 vorliegen. Diese Komplexierung, beispielsweise bei Am³⁺, ist so stark, daß eine nahezu vollständige Extraktion in eine organische Phase erfolgt, die Calix[6]arene gelöst in einem Verdünnungsmittel enthält. Alternativ dazu können die Calix[6]arene ungelöst als Feststoff eingesetzt werden, wobei es seine Selektivität beibehält.

## Beschreibung

Die Erfindung betrifft neue Calix[6]arene, ein Verfahren zu ihrer Herstellung und ihre Verwendung.

Durch Ludwig, R. ist in Fresenius' J. Analyt. Chem. 2000, Bd. 367, S. 103-128, beschrieben worden, dass substituierte [1₆]Metacyclophane als Calix[6]arene bezeichnet werden. Sie sind eine Klasse von Calixarenen. Daneben existieren andere mit n = 4 bis über 8. Sie werden in einfacher Reaktion aus Phenol und

Formaldehyd hergestellt und lassen sich in ihren Substituenten vielfältig variieren.

Durch Ludwig, R.; Kunogi, K.; Nguyen, T. K. D.; Tachimori, S. wurde in J. Chem. Soc., Chem. Commun. 1997, S. 1985 -1986, bekannt, dass Calix[6]arene mit der Strukturformel 1 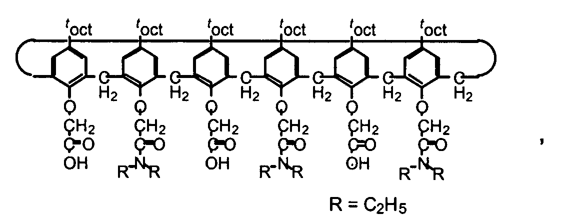 die substituierte Carbonsäureamide tragen, aus schwach sauren Lösungen dreiwertige Actinide selektiv extrahieren. Nachteilig ist jedoch, dass die Lösungsmittelextraktion erst bei pH = 3 quantitativ abläuft.

In Solvent Extraction Research and Developm. Jpn. 2001, Bd. 8, S. 159-164, schlagen Hoshi, H.; Tsuyoshi, A. und Akiba, K. substituierte Dithiophosphinsäuren zur Abtrennung dreiwertiger Actiniden vor. Dabei hat sich jedoch bei dem chemischen Trennverfahren die unzureichende chemische Stabilität der Dithiophosphinsäuren als problematisch erwiesen.

Delmau, L. H.; Simon, N. et al. beschreiben in J. Chem. Soc., Chem. Commun. 1998, S. 1627-1628, Calix[4]arene mit Carbamoylmethylphosphinoxid- (CMPO-) Gruppen zur Abtrennung dreiwertiger Actiniden. Es hat sich aber als nachteilig erwiesen, dass die Trennfaktoren, also die Wirksamkeit der Trennung, niedrig und die Synthese aufwendig sind.

Kolarik, Z.; Müllich, U.; Gassner, F. in Solv. Extr. Ion Exchange, Bd. 17, S. 1155 ff., verwenden Pyridin-Abkömmlinge zur Abtrennung dreiwertiger Actiniden. Der Nachteil dieser Mittel besteht in der sehr langsamen Einstellung des chemischen Gleichgewichts, also in einer langsamen Kinetik, so dass der technologischer Prozeß mit zu langen Verweilzeiten verbunden ist.

Durch EP 0 883 601 ist bekannt, dass Calix[4]arene mit je einer Carbonsäuregruppe und Carbonsäureamidgruppe und zwei freien OH-Gruppen aus sauren Lösungen dreiwertige Actinide nicht selektiv von einem Überschuß an Lanthaniden abtrennen können.

Nach Ludwig. R. et al., in Proc. Sympos. Solvent Extr., Fukuoka, Japan, S. 41-42; Solv. Extr. Research and Developm. Jpn., 1996, Bd. 3, S. 244- 254, können solche Calix[4]arene mit einer Kombination aus Carbonsäuregruppe und Carbonsäureamidgruppen Metallionen mit hohen Koordinationszahlen wie Ln(III) und An(III) an beispielsweise Am, Cm aus technisch relevanten Lösungen mit pH-Wert < 3 weder binden und entfernen, noch eine Gruppentrennung zwischen den chemisch sehr ähnlichen Lanthaniden und Actiniden erreichen.

In EP 0 883 601 wird ein pH-Wert von > 5 angegeben, um eine hinreichende, aber noch nicht quantitative Extraktion zu erreichen. Das Einstellen eines solch hohen pH-Wertes ist bei einer technischen Anwendung nicht sinnvoll, da beim Abstumpfen saurerer Lösungen große Mengen an Salz als Sekundärabfall entstehen.

Die Calix[6]arene tragen einen genügend hohe Anzahl von Donoratomen/Donorgruppen für dreiwertige f-Elemente mit Koordinationszahlen von 8 bis 9, was bei Calix[4]arenen mit einzähnigen Substituenten nicht der Fall ist. Als Donoratome müssen bei den Liganden die phenolischen OH-Gruppen in deprotonierter Form wirken, daher eignen sich die Liganden nicht zur Extraktion aus sauren Lösungen.

Die in JP 05320092A beschriebenen Calix[6]arene wirken zwar zur Extraktion von Ln(III), aber nicht zur Ln(III)/An(III)-Gruppentrennung und nicht zur Extraktion aus stark sauren Lösungen.

Der Erfindung liegt die Aufgabe zugrunde, geeignete Calix[6]arene zu schaffen, die Nachteile der bisher vorgeschlagenen Lösungen zu beseitigen.

Die Aufgabe der Erfindung wird durch Calix[6]arene gelöst, die alpha-halogenierte Carbonsäuregruppen enthalten und die Struktur entsprechend der der Strukturformel 2 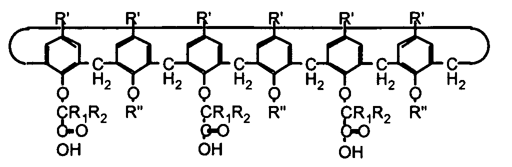 besitzen.

Dabei sind
R₁ = F, Cl oder Br,
R₂ = H, F, Cl oder Br,
R' = tertiär-Butyl, tertiär-Octyl, H, Aryl- oder eine andere Alkylgruppe,
R" = H, Alkyl-, Aryl-, Alkylcarbonylmethoxy-, NH- oder N, N-disubstituierte Carbamoylmethoxy-Gruppe.

Die erfindungsgemäßen Calix[6]arene sind Verbindungen, welche verschiedene funktionelle Gruppen, unter ihnen aber immer alpha-halogenierte Carbonsäuren, tragen.

Diese Calix[6]arene sind nichttoxisch und vollständig unlöslich in der wäßrigen Phase. Als Feststoffe sind sie schwer entflammbar und unbegrenzt lagerbar.

Die Synthese führt bei den erfindungsgemäßen Calix[6]arenen infolge eines Templat-Effektes direkt zu dreifach substituierten Derivaten mit der gewünschten Struktur. Der Templat-Effekt kommt zustande durch
a) den Einsatz von Cs₂CO₃ oder K₂CO₃ oder ähnlichen zur schrittweisen Deprotonierung der OH-Gruppen des Precursors und
b) die Größe der Halogenatome, die eine Weiterreaktion zu 4-, 5- oder 6-fach mit COOR substituierten Calix[6]arenen infolge sterischer Hinderung vermeiden.

Das ist ein Vorteil gegenüber bisher bekannten Synthesen mit R1=R2=H in Grundformel der erfindungsgemäßen Calix[6]arenen, da das dreifach substituierte Derivat nur im Gemisch mit höher substituierten Derivaten in einer kinetisch kontrollierten Reaktion entsteht.

Bei bestimmten Synthesebedingungen entstehen Nebenprodukte mit der Struktur

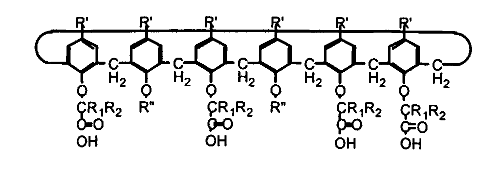

Die erfinungsgemäßen Calix[6]arene lassen sich in zwei Synthesestufen in guten chemischen Ausbeuten herstellen. Sie sind chemisch und radiolytisch außerordentlich stabil, was sich darin äußert, daß sie ohne Einbuße an Trennwirkung mehrfach im Kreislauf Extraktion/Rückextraktion gefahren werden können.

Das erfindungsgemäße Verfahren zur Herstellung der erfinungsgemäßen Calix[6]arene, die alpha-halogenierte Carbonsäuregruppen enthalten, besteht darin, dass ein substituiertes Phenol, beispielsweise tertiär-Butylphenol oder tert.-Octylphenol, mit Paraformaldehyd und Kalilauge in Xylen zum Calix[6]aren mit der Strukturformel 3 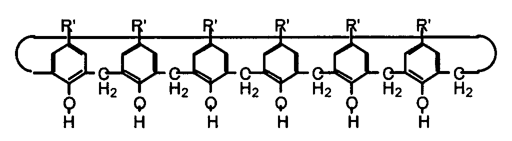 kondensiert wird. Dieses bekannte Calix[6]aren mit der Strukturformel 3 wird mit einer Base, beispielsweise Cs₂CO₃ oder K₂CO₃, einem alpha-halogenierten Carbonsäure- ester, beispielsweise BrCF₂COOC₂H₅ und einem Aktivator, beispielsweise Kaliumjiodid, in einem aprotischen Lösungsmittel umgesetzt. Als Lösungsmittel kann beispielsweise Tetrahydrofuran verwendet werden. Bei der Reaktion deprotoniert zuerst die Base das Phenol, woraufhin der Ester am O⁻ angreift. Die Rolle des Hilfsstoffes besteht in einem in-situ Halogenaustausch zur Erhöhung der Reaktivität. Als Base können auch andere Verbindungen, beispielsweise NaH, KH, eingesetzt werden, wobei jedoch die Ausbeute infolge von Nebenreaktionen am halogenierten Ester sinkt. Als Halogen sind F, Cl und Br geeignet. Die besten Anwendungseigenschaften ergeben sich mit R₁ = R₂ = F. Das Produkt ist ein intermediärer Ester, der mit Natronlauge hydrolysiert wird. Dieses entsteht in quantitativer Ausbeute von Calix[6]arenen, die alpha-halogenierte Carbonsäuregruppen gemäß Grundformel mit R" = H enthalten. Die erfindungsgemäßen Calix[6]arene mit der Strukturformel 2 eignen sich als Komplexbildner, vor allem zur Komplexierung dreiwertiger Actiniden und deren Trennung von anderen Metallionen sowie Matrixbestandteilen. Die erfindungsgemäßen Calix[6]arene binden die dreiwertigen Actinide besser als die Lanthanide. Deshalb eignen sie sich besser zur Extraktion und zur Gruppentrennung.

Die chemische Trennung der dreiwertigen Actiniden von Lanthaniden zählt wegen der ähnlichen chemischen Eigenschaften der Metallionen zu den schwierigsten Trennproblemen in der Chemie überhaupt.

Überraschender Weise hat sich herausgestellt, dass die Synthese zu partiell substituierten Calix[6]arenen führt und die erfindungsgemäßen Calixarene auch bei pH-Werten kleiner als 4 dreiwertige Actiniden sehr gut extrahieren können. Diese Eigenschaft kommt dadurch zustande, dass anstelle der zu kleinen Struktur des bekannten Calix[4]arens ein Calix[6]aren-Grundgerüst vorhanden ist, bei dem das Metallion tiefer in den makrozyklischen Hohlraum eindringen kann.

Dieser Hohlraum enthält alpha-basische Phenylgruppen, die mit Actiniden infolge des stärkeren relativistischen Effektes besser in Wechselwirkung treten als mit Lanthaniden. Dadurch kommt eine Gruppentrennung An(III) über Ln(III) zustande. Die bekannten Calix[4]arene sind dazu nicht in der Lage.

Wird eine Anwendung zur Reinigung sehr stark saurer wäßriger Phasen verlangt, so kann R" durch Carbonsäureester oder -amid-Gruppen substituiert werden. Dabei verstärkt sich der makrozyklische Effekt und das Actinidion wird stärker komplexiert.

Zur Anwendung wird eine Verbindung mit der Strukturformel 2 in einem organischen Verdünnungsmittel gelöst und mit einer wäßrigen Lösung in Kontakt gebracht, die außer Actiniden(III) auch einen großen Überschuß an Salzen anderer Metalle wie Lanthaniden, Alkali, Erdalkali und Mineralsäuren enthalten kann. Die Verbindungen extrahieren mit hoher Selektivität die Actiniden(III) und belassen die anderen Salze und Säuren in der wäßrigen Phase. Die Selektivität gründet sich auf die käfigförmige Struktur der Verbindung und der Substituenten. Aus der beladenen organische Phase werden die Actiniden duch Kontaktierung mit konzentrierterer Mineralsäure zurückgewonnen. Dabei wird das Extraktionsmittel in die ursprüngliche, unkomplexierte Form überführt und steht für einen neuen Zyklus zur Verfügung.

Die Anwendungen solcher Trennungen sind:
a) Analytik von radioaktiven Abfällen, wenn diese Alphastrahler enthalten und die Alphastrahler keine genügend intensive Gammastrahlung emittieren. Die Bewertung des Abfallgemisches erfordert die Abtrennung dieser Alphastrahler von der Matrix. Bei den Alphastrahlern handelt es sich um Actinide, insbesondere Transplutoniumelemente (TPU's), die in dreiwertigem Zustand vorliegen.
b) Abtrennung der Transplutoniumelemente in einem 'advance nuclear fuel cycle'. Die abgetrennten Elemente Am + Cm werden anschließend durch Transmutation verbrannt. Dadurch wird die Langzeit-Sicherheit von Kernbrennstoff-Abfalllagern erhöht und das Risiko deutlich verringert.

Vorteile gegenüber bisherigen chemischen Trennungen dreiwertiger Actinide von Lanthaniden und anderen Metallsalzen sind:
Der Vorteil der Anwendung des erfindungsgemäßen Calix[6]arens ist eine Abtrennung aus stark sauren wäßrigen Lösungen. Dies wird durch die höhere Acidität der sauren Gruppen des erfindungsgemäßen Calix[6]arens erreicht.

Im Gegensatz zu den herkömmlichen Dithiophosphinsäuren sind die erfindungsgemäßen Calix[6]arene chemisch sehr stabil, wasserunlöslich auch im neutralen Medium und toxikologisch unbedenklich.

Im Gegensatz zu CMPO-Derivaten zeichnet sich die erfindungsgemäßen Calix[6]arene durch bedeutend höhere Trennfaktoren aus, die eine Trennung wirtschaftlich und risikoarm ermöglichen. Die Trennfaktoren ergeben sich aus der Struktur des Liganden und sind bei den erfindungsgemäßen Calix[6]arenen besonders hoch. Die Struktur wird durch den Templateffekt bei der Synthese erreicht.

Die Erfindung soll an einem Ausführungsbeispiel erläutert werden.

Zur Herstellung des erfindungsgemäßen Calix[6]arens mit R_{1,2} = F, R' = tert-Butyl, R" = OH werden folgende Synthesebedingungen angewandt:
6 Gramm tert-Butylcalix[6]aren mit der Strukturformel 3 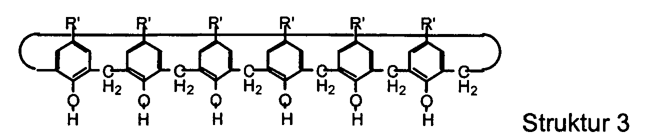 werden mit 30 Gramm Cäsiumcarbonat, 6 Gramm Kaliumiodid und 9 Milliliter Bromdifluoressigsäureethylester in Tetrahydrofuran / 5% Dimethylformamid 10 Tage lang unter Stickstoff am Rückfluß erhitzt.

Nach Abkühlen wird filtriert, das Filtrat eingeengt, nach Zugabe von CH₂Cl₂ mit HCI neutralisiert, mehrmals mit verdünnter HCI gewaschen, eingeengt und durch Zugabe von Ethanol zur Kristallisation gebracht. Der getrocknete Niederschlag besteht aus dem Ester-Vorläufer des erfindungsgemäßen Calix[6]arens mit COOC₂H₅ anstelle von COOH. Er wird in THF gelöst und mit Natronlauge durch 12-stündiges Erhitzen am Rückfluß hydrolysiert. Die organische Phase wird eingeengt, nach Zugabe von CH₂Cl₂ mit HCI neutralisiert und mehrmals mit verdünnter HCI gewaschen. Eindampfen der organischen Phase ergibt das erfinungsgemäße Calix[6]aren der Struktur 2 mit R_{1,2} = F, R' = tert-Butyl, R" = OH.

Anstelle von Cäsiumcarbonat kann Kaliumcarbonat verwendet werden, insbesondere beim scale-up der Synthese. Anstelle von Bromdifluoressigsäureethylester kann Bromfluoressigsäureethylester eingesetzt werden, wobei erfindungsgemäße Calix[6]aren mit R₁ = F, R₂ = H entsteht. Anstelle der fluorierten Ester können die chlorierten oder bromierten Ester eingesetzt werden, wobei Produkte mit R_{1,2} = Cl oder Br bzw. R₁ = Cl oder Br und R₂ = H entstehen. Die Extrahierbarkeit von dreiwertigen Actiniden verschiebt sich bei R₂ = H oder bei R₁ = Cl oder Br zu etwas höheren pH-Werten, was durch die Säurestärken der Substituienten am Calixaren verursacht wird.

Die Produkte wurden anhand ihrer NMR-Spektren, IR-Spektren, Massenspektren, Elementaranalysen und Chromatogramme nachgewiesen: z. B. CF₂COOCH₂CH₃ im 1H-NMR bei 4.4 ppm, nach Hydrolyse zu CF2COOH abwesend, Integral im 1H-NMR zeigt Dreifach-Substituierung, 13.78 und 63.76 im ¹³C-NMR zeigt Ethylgruppe in CF₂COOCH₂CH₃, die nach Hydrolyse abwesend ist, 117 ppm Triplet (starke C-F-Kopplung) im ¹³C-NMR, CF₂ bei -78 ppm und CHF bei ―129 ppm im ¹⁹F-NMR, CF₂COOR Carbonylschwingung bei 1778 cm⁻¹, gegebenenfalls außerdem CH₂C(O)N(C₂H₅)₂ Carbonyl bei 1675 cm⁻¹ im IR, FAB(⁺) des erfindungsgemäßen Calix[6]arens mit R_{1,2} = F, R' = tert-Butyl, R" = OH m/z=1363 (2 · Na⁺).

Die Anwendung des erfindungsgemäßen Calix[6]arens, das alpha-halogenierte Carbonsäuregruppen enthält und mit R_{1,2} = F, R' = tert-Butyl, R" = OH, ergeben sich bei der Flüssig-flüssig-Extraktion folgende Verteilungskoeffizienten D (D = Konzentration Metall in der org. Phase / Konz. Metall in der wäßrigen Phase):

| pH | D (Am) | (Eu) |
|---|---|---|
| 3,0 | 190,5 | 6,6 |
| 2,4 | 5,4 | 0,39 |

Versuchsbedingungen: 1 millimolare Lösung Ligand in Chloroform, trägerfreies Am-241, 0.1 millimolare Lösung von Europium, Salpetersäure.

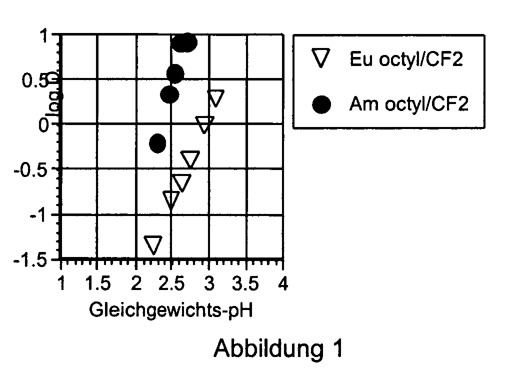

In Abbildung 1 ist der Logarithmus der Verteilungskoeffizienten von Eu(III) (0.05 millimolar) und Am(III) (Am-241 trägerfrei) bei der einstufigen Extraktion mit 3 millimolarer Lösung an (2) mit R_{1,2} = F, R' = tert-Octyl, R" = OH in CHCl₃ aus Salpetrersäure dargestellt.

Durch eine Erhöhung der Ligandkonzentration kann die Extraktionsausbeute bei niedrigen pH-Werten in einfacher Weise gesteigert werden.

Die Trennfaktoren einer einstufigen Extraktion/Reextraktion sind somit größer als 10 und eine effektive Trennung wird erreicht.

Die Vorteile des erfindungsgemäßen Calix[6]arens sind die Abtrennung aus stark sauren wäßrigen Lösungen, nachweisbar in Laborversuchen, die Kreislaufführung des Prozesses, nachweisbar durch mehrfaches Rezyklisieren im Labormaßstab, die Wasserunlöslichkeit, nachweisbar anhand von UV-Spektren, die toxikologische Unbedenklichkeit infolge der Wasserunlöslichkeit und eines Dampfdruckes von null, die chemische und radiolytische Stabilität, nachweisbar durch Aufnahme von Spektren nach mehrmaliger Anwendung und die hohen Trennfaktoren gegenüber störenden Metallsalzen und Säuren.

## Patentansprüche

1. Calix[6]arene, **dadurch gekennzeichnet, daß** sie alpha-halogenierte Carbonsäuregruppen enthalten.

2. Calix[6]arene nach Anspruch 1, **dadurch gekennzeichnet, dass** sie die Struktur der Formel 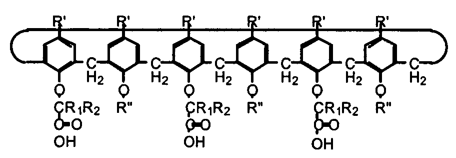 mit
R₁ = F, Cl oder Br,
R₂ = H, F, Cl oder Br,
R' = tertiär-Butyl, tertiär-Octyl, H, Aryl- oder eine andere Alkylgruppe,
R" = H, Alkyl-, Aryl-, Alkylcarbonylmethoxy-, NH- oder N, N-disubstituierte Carbamoylmethoxy-Gruppen besitzen.

3. Calix[6]arene nach Anspruch 1 und 2, **dadurch gekennzeichnet, dass** die Verbindungen aus
R' = tertiär-Butyl, R₁ = R₂ = F, R" = H oder
R' = tertiär-Octyl, R₁ = R₂ = F, R" = H oder
R' = tertiär-Octyl, R₁ = F, R₂ = H, R" = H oder
R' = tertiär-Butyl, R₁ = F, R₂ = H, R" = H oder
R' = tertiär-Butyl, R₁ = R₂ = F, R" = CH₂C(O)N(C₂H₅)₂ oder
R' = tertiär-Butyl, R₁ = R₂ = Cl, R" = H
bestehen.

4. Calix[6]arene nach Anspruch 1 bis 3, **dadurch gekennzeichnet, dass** bei bestimmten Synthesebedingungen Nebenprodukte mit der Struktur 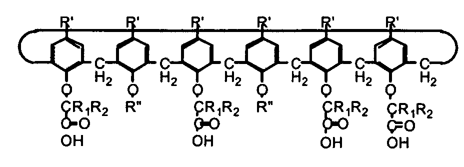 entstehen.

5. Verfahren zur Herstellung von Calix[6]arenen nach Anspruch 1 bis 4, **dadurch gekennzeichnet, dass** unter Anwendung einer Base, bestehend aus zwei Äquivalenten je anfänglicher OH-Gruppe Cs₂CO₃, K₂CO₃, NaH oder andere, und eines Precursors, bestehend aus drei Äquivalenten BrCR₁R₂COOR* je Calixaren-Molekül, R₁ = F, Cl, Br, R₂ = H, F, Cl, Br, R* = C₂H₅, Alkyl, Aryl und einem Hilfsstoff Kl, Nal, der den halogenierten Precursor intermediär iodiert, in THF mit einem Zusatz von DMF unter N₂ am Rückfluß erhitzt wird und dass die Aufarbeitung durch Filtrieren, Einengen, Neutralisieren, Waschen, und Auskristallisieren aus Ethanol und Hydrolysieren erfolgt und dass bei der Hydrolyse durch Kochen mit Natronlauge oder anderer Lauge R* in H überführt wird.

6. Calix[6]arenen nach Anspruch 1 bis 4, **dadurch gekennzeichnet, dass** sie als Komplexbildner verwendbar sind.

7. Calix[6]arenen nach Anspruch 1 bis 4 und 6, **dadurch gekennzeichnet, dass** sie zur Extraktion dreiwertiger Actinide verwendet werden.

8. Calix[6]arenen nach Anspruch 1 bis 4 und 6, **dadurch gekennzeichnet, dass** sie dreiwertige Actinide selektiv gegenüber Lanthaniden-, Alkali- und Alkalisalzen komplexieren und binden und daß die Selektivität zur Abtrennung mittels Flüssig-flüssig-Extraktion oder Fest-flüssig-Extraktion genutzt wird.

9. Calix[6]arenen nach Anspruch 1 bis 4 und 6, **dadurch gekennzeichnet, dass** zur Erhaltung ihrer chemischen Eigenschaften der Komplexbildner zur Adsorption dreiwertiger Actinide auf einen festen Träger fixiert ist oder der Komplexbildner zur Herstellung eines Co-Polymerisates oder als Feststoff direkt eingesetzt wird.

10. Calix[6]arenen nach Anspruch 1 bis 4 und 6, **dadurch gekennzeichnet, dass** die Trennung von Actiniden aus sauren, salzhaltigen Matrices in einer Austauschersäule erfolgt.
